(19) **Europäisches Patentamt / European Patent Office / Office européen des brevets**

(11) **EP 4 678 759 A1**

(12) **EUROPEAN PATENT APPLICATION**
published in accordance with Art. 153(4) EPC

(43) Date of publication:
**14.01.2026 Bulletin 2026/03**

(21) Application number: **24807600.2**

(22) Date of filing: **17.05.2024**

(51) International Patent Classification (IPC):
*C12P 13/00* (2006.01)     *C12P 13/08* (2006.01)
*C12N 15/77* (2006.01)     *C12N 9/88* (2006.01)

(52) Cooperative Patent Classification (CPC):
**C12N 9/88; C12N 15/77; C12P 13/00; C12P 13/08**

(86) International application number:
**PCT/KR2024/006740**

(87) International publication number:
**WO 2024/237729 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:  **18.05.2023  KR 20230064325**

(71) Applicant: **CJ Cheiljedang Corporation**
**Seoul 04560 (KR)**

(72) Inventors:
• **SHIN, Ji Hyun**
  **Seoul 04560 (KR)**
• **PARK, Sang Min**
  **Seoul 04560 (KR)**
• **KWAK, Dong Hun**
  **Seoul 04560 (KR)**

• **LEE, Su Jin**
  **Seoul 04560 (KR)**
• **PARK, Jeong Ho**
  **Seoul 04560 (KR)**
• **NOH, Seung-woo**
  **Seoul 04560 (KR)**
• **KIM, Jun-Woo**
  **Seoul 04560 (KR)**
• **LEE, Kang Hoon**
  **Seoul 04560 (KR)**
• **LEE, Hanhyoung**
  **Seoul 04560 (KR)**
• **SEO, Chang Il**
  **Seoul 04560 (KR)**

(74) Representative: **Meissner Bolte Partnerschaft mbB**
**Patentanwälte Rechtsanwälte**
**Postfach 86 06 24**
**81633 München (DE)**

(54) **PROCESS FOR PREPARING CADAVERINE ADIPATE**

(57)     The present disclosure relates to a method for preparing cadaverine adipate, comprising: a first step of obtaining lysine adipate by culturing an L-lysine-producing microorganism in a medium to which adipate is added in the form of diammonium adipate; and a second step of converting lysine adipate into cadaverine adipate. According to the present disclosure, cadaverine adipate can be obtained with high purity by performing the process according to the above-described procedure, without an ion resin exchange, a decarbonation process, and a distillation process.

[FIG. 1]

Start

Obtaining lysine adipate by culturing L-lysine-producing microorganism in a medium containing diammonium adipate

Converting obtained lysine adipate into cadaverine adipate

End

**Description**

**[Technical Field]**

The present disclosure relates to a technique for preparing cadaverine adipate with high purity using microbial fermentation and a purification process.

**[Background Art]**

Cadaverine is a foul-smelling, toxic diamine compound produced by the decomposition of animal tissues. Additionally, cadaverine adipate is a precursor for polymerization of bio-polyamides, and is one of the engineering plastics widely used in automobiles, electrical and electronic components, *etc.*

The preparation process for cadaverine adipate generally includes the following steps:
Synthesis of Cadaverine: Cadaverine synthesis involves the fermentation of an appropriate microbial strain capable of producing lysine decarboxylase, an enzyme that catalyzes the decarboxylation of lysine to cadaverine.

Preparation of Adipic Acid: Adipic acid, another monomer required for the production of cadaverine adipate, can be synthesized by various methods, such as the oxidation of cyclohexene or cyclohexanol using nitric acid or air.

Formation of Cadaverine Adipate: The formation of cadaverine adipate involves the reaction between cadaverine and adipic acid in the presence of a suitable catalyst such as sulfuric acid or phosphoric acid. The reaction typically involves mixing the two monomers at an appropriate temperature and stirring for a certain period of time to ensure complete reaction.

Purification of Cadaverine Adipate: Once the reaction is complete, cadaverine adipate, the raw material, is purified using various separation and purification techniques, such as solvent extraction, chromatography, and crystallization, *etc.*

According to the prior art, since adipate has low solubility, there was a problem that adipate precipitated as crystals together with cadaverine adipate during the crystallization process. Accordingly, in order to produce high purity cadaverine adipate, the adipate had to be removed before crystallization, and there were problems of cost burden associated with performing an additional process to remove the adipate, reduced process efficiency, and environmental pollution due to the use of chemical solvents, *etc.*

**[Disclosure]**

**[Technical Problem]**

The technical problem to be solved herein relates to a method for preparing cadaverine adipate.

**[Technical Solution]**

The present disclosure provides a method by which cadaverine adipate can be prepared with high purity by adding diammonium adipate to an L-lysine-producing microorganism and culturing the same, without the conventional ion resin exchange, decarbonation process, or distillation process, in the preparation of cadaverine adipate.

**[Advantageous Effects]**

According to one aspect of the present disclosure, lysine adipate is obtained by culturing an L-lysine-producing microorganism in a medium containing diammonium adipate, and lysine adipate is enzymatically converted into cadaverine adipate, and thus, high purity cadaverine adipate can be prepared at high yield, since there is no need for a separate process to remove adipate, thereby simplifying the process, and adipate crystals do not precipitate together as a by-product.

**[Brief Description of Drawings]**

FIG. 1 is a flow chart showing the process for preparing cadaverine adipate according to the present disclosure.
FIG. 2 is a flowchart showing the process for preparing cadaverine adipate according to one aspect of the present disclosure.
FIG. 3 is a graph showing the results of converting lysine adipate to cadaverine adipate using strains expressing lysine decarboxylase.

[Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below.

One aspect of the present disclosure provides a method for preparing cadaverine adipate, comprising: a first step of obtaining lysine adipate by culturing an L-lysine-producing microorganism in a medium to which adipate is added in the form of diammonium adipate; and a second step of converting lysine adipate into cadaverine adipate.

As used herein, the term "cadaverine (CAD)" is a foul-smelling, toxic diamine compound and has the chemical formula of $NH_2(CH_2)_5NH_2$. Cadaverine may also be named 1,5-pentanediamine or pentamethylenediamine. In the present disclosure, it should be understood that cadaverine comprises the cadaverine adipate form.

As used herein, the term "cadaverine adipate" refers to a substance prepared by reacting cadaverine in the form of diamine with adipic acid, a dicarboxylic acid component, and has high industrial utility as a preparation precursor for polyamide resins.

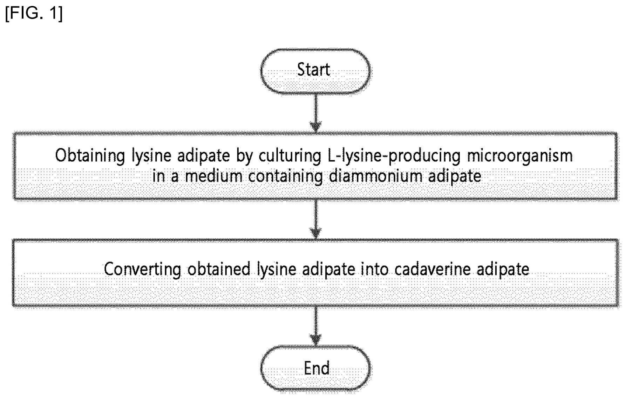

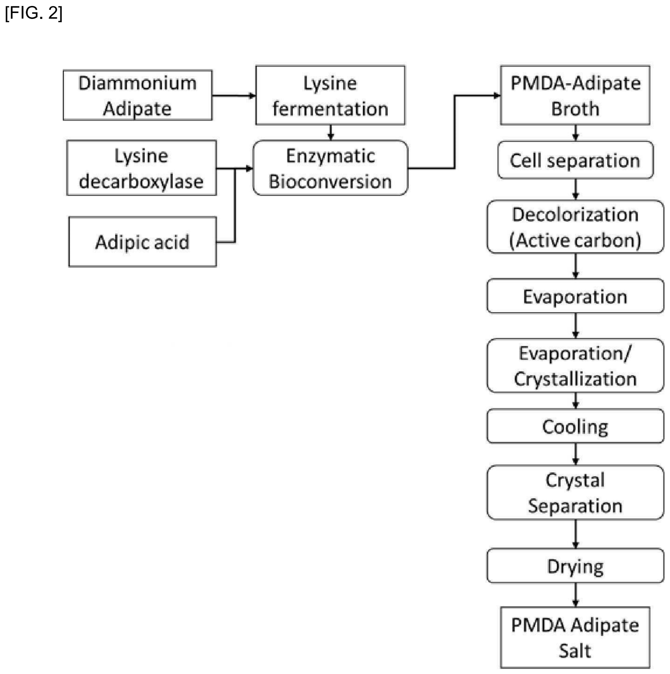

FIGS. 1 and 2 are flowcharts showing the process for preparing cadaverine adipate according to one aspect of the present disclosure.

As used herein, the term "L-lysine-producing microorganism" may comprise all wild-type microorganisms, or naturally or artificially genetically modified microorganisms, and it may be a microorganism in which a particular mechanism is weakened or enhanced due to the insertion of a foreign gene, or enhancement or inactivation of the activity of an endogenous gene, *etc*., and may be a microorganism comprising genetic modification to produce L-lysine

In one example, the L-lysine-producing microorganism may be a microorganism naturally having the L-lysine-producing ability, a microorganism, in which the L-lysine-producing ability has been imparted to a microorganism having no L-lysine-producing ability, or a microorganism in which the L-lysine-producing ability has been enhanced in a microorganism having significantly low L-lysine-producing ability, but is not limited thereto. Specifically, as used herein, the L-lysine-producing microorganism or the microorganism having an L-lysine-producing ability may be a microorganism in which some genes in the biosynthesis pathway of L-lysine are enhanced or weakened, or some genes in the degradation pathway of L-lysine are enhanced or weakened. The "enhancement" or "increase" in the L-lysine-producing ability may mean that the L-lysine-producing ability is enhanced compared to that of a parent strain or a non-modified microorganism.

In a specific example, the microorganism may be a microorganism of the genus *Corynebacterium* or the genus *Escherichia.* The microorganism of the genus *Corynebacterium* may comprise all microorganisms belonging to the genus *Corynebacterium.* Specifically, it may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* more specifically, *Corynebacterium glutamicum,* but is not limited thereto. Additionally, the microorganism of the genus *Escherichia* may comprise all microorganisms belonging to the genus *Escherichia.* Specifically, it may be *Escherichia coli, Escherichia albertii, Escherichia blattae, Escherichia fergusonii, Escherichia hermannii,* or *Escherichia vulneris,* more specifically, the microorganism of the genus *Escherichia* may be *Escherichia coli,* but is not limited thereto.

The cultivation of the L-lysine-producing microorganism may be performed in a suitable culture medium and culture conditions known in the art. Such a culturing process may be easily adjusted for use by those skilled in the art according to the microorganism to be selected. Specifically, the cultivation may be a batch culture, a continuous culture, or a fed-batch culture, but is not limited thereto.

As used herein, the term "medium" refers to a mixture of materials which contains nutrient materials required for the culturing of the microorganism as a main ingredient, and it supplies nutrient materials and growth factors, along with water that is essential for survival and growth. Specifically, the medium used for culturing the L-lysine-producing microorganism of the present disclosure may be any medium used for conventional cultivation of microorganisms without any particular limitation, except that it contains diammonium adipate as part or all of the nitrogen source. The microorganism of the present disclosure may be cultured under aerobic conditions in a conventional medium containing a nitrogen source including diammonium adipate, an appropriate carbon source, phosphorus source, inorganic compound, amino acid, and/or vitamin, *etc.,* while adjusting temperature, pH, *etc.*

As used herein, the term "adipic acid" is a dicarboxylic acid with 6 carbon atoms and has the chemical formula of $(CH_2)_4(COOH)_2$, and generally exists as white crystals or crystalline powder. The adipic acid is also named hexanedioic acid, *etc.,* and is a compound with very high industrial utility as a precursor for producing nylon.

The first step in the method for preparing cadaverine dicarboxylate of the present disclosure is a step of obtaining lysine adipate by culturing an L-lysine-producing microorganism in a medium to which adipate is added in the form of diammonium adipate. This step may be carried out via a fermentation process of an L-lysine-producing microorganism.

In the first step, the adipate may be supplied to the medium in the form of diammonium adipate, and accordingly, in the present disclosure, it should be understood that adipate comprises the diammonium adipate form.

As used herein, the term "diammonium adipate" is a form in which two hydrogen atoms of adipic acid are substituted with ammonium, and is used as the main nitrogen source of the medium. The diammonium adipate may be prepared by gently mixing adipic acid with ammonia water to have a neutral pH, followed by concentration to be used in crystal form or liquid form, but is not limited thereto.

The preparation method of the present disclosure enables the preparation of cadaverine adipate with high purity, while simplifying the process by adding 56 mol% or more of adipate supplied in the process in the form of diammonium adipate to the medium in the first step, and to reduce the side effect that adipate precipitates together with cadaverine adipate.

Specifically, diammonium adipate in the first step may be contained in the medium at a high ratio, specifically 81 mol% or more of the adipate supplied during the entire process of the preparation method of the present disclosure.

More specifically, the diammonium adipate in the first step may be contained in an amount of 56 mole% or more, 66 mole% or more, 74 mole% or more, 81 mole% or more, 82 mole% or more, 83 mole% or more, 84 mole% or more, 85 mole% or more, 86 mole% or more, 87 mole% or more, 88 mole% or more, 89 mole% or more, 90 mole% or more, 91 mole% or more, 92 mole% or more, 93 mole% or more, 94 mole% or more, 95 mole% or more, 96 mole% or more, 97 mole% or more, 98 mole% or more, 99 mole% or more, or 100 mole% of the adipate supplied during the entire process of the preparation method of the present disclosure.

The preparation method of the present disclosure may provide advantages in that cadaverine adipate can be prepared with high purity by containing a large amount of diammonium adipate in the medium, without performing a separate removal step of adipate, such as ion resin exchange, decarbonation process, or distillation process, *etc.,* unlike the conventional process of adding ammonium sulfate, *etc.* Additionally, the molar ratio of adipate added during the entire process/cadaverine in the process liquid after the second step may be easily adjusted using diammonium adipate.

Specifically, the diammonium adipate in the first step may be contained so that the molar ratio (diammonium adipate/lysine) relative to lysine to be produced is 0.55 to 1. More specifically, the diammonium adipate in the first step may be contained so that the molar ratio (diammonium adipate/lysine) relative to lysine to be produced is 0.65 to 0.98, 0.73 to 0.98, or 0.85 to 0.98, more specifically, 0.88 to 0.96. In particular, it should be understood that the amount of lysine to be produced means the total amount comprising all lysine adipate forms.

In one example, the diammonium adipate in the first step may be contained so that the molar ratio (diammonium adipate/lysine) relative to lysine to be produced is in the range consisting of one lower limit selected from 0.55, 0.60, 0.65, 0.7, 0.85, 0.86, 0.87, 0.88, 0.89, 0.9, 0.91, and 0.92 and/or one upper limit selected from 1, 0.99, 0.98, 0.97, 0.96, 0.95, 0.94, 0.93, and 0.92.

Specifically, the diammonium adipate in the first step may be contained in an amount of 70 g/L to 140 g/L based on the fermentation liquid of the L-lysine-producing microorganism. More specifically, the diammonium adipate in the first step may be contained in an amount of 80 g/L to 130 g/L, 90 g/L to 130 g/L, 105 g/L to 130 g/L, or 105 g/L to 120 g/L based on the fermentation liquid of the L-lysine-producing microorganism. When the diammonium adipate is added at the above concentration, the ammonium ion acts as a nitrogen source during fermentation, and adipate and lysine form salts, and thus can be contained in a dissolved state in the fermentation liquid.

Lysine adipate can be produced through fermentation of the L-lysine-producing microorganism in a medium containing diammonium adipate. Lysine adipate can be added to the subsequent process as it is, in the form of a fermentation liquid from which the microbial cells are removed, or after concentration or purification.

The concentration of lysine in the fermentation liquid, in which the L-lysine-producing microorganism of the present disclosure is cultured in the first step, is not limited, but may be in the range of 90 g/L to 300 g/L, 95 g/L to 250 g/L, 97 g/L to 160 g/L, or 97 g/L to 100 g/L, based on the fermentation liquid of the L-lysine-producing microorganism.

The carbon source of the medium may include carbohydrates, such as glucose, fructose, sucrose, maltose, and their isomers, *etc.*; sugar alcohols, such as mannitol, sorbitol, *etc.*; organic acids, such as pyruvic acid, lactic acid, citric acid, *etc.*; amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e.,* molasses converted to reducing sugar) may be used, and in addition, various other carbon sources in an appropriate amount may be used without limitation. These carbon sources may be used alone or in a combination of two or more kinds, but are not limited thereto.

In a specific example, the carbon source that may be included in the medium of the present disclosure may include glucose, maltose, or maltose isomers. More specifically, it may include one or more selected from glucose, maltose, and maltose isomers (isomaltose), or a combination of two or more thereof, but is not limited thereto.

The phosphorous source of the medium may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.*

Additionally, the culture medium may include metal salts, such as magnesium sulfate or iron sulfate needed for growth. Lastly, the culture medium may include essential growth substances such as amino acids and vitamins, in addition to the above substances. Further, the culture medium may include appropriate precursors. These substances may be added to the culture in an appropriate manner, *e.g.,* in a batch culture or continuous culture, but are not limited thereto.

The nitrogen source of the medium may include diammonium adipate. The nitrogen source of the conventional medium for culturing microorganisms may include inorganic nitrogen sources, such as ammonia, ammonium sulfate, ammonium chloride, ammonium acetate, ammonium phosphate, ammonium carbonate, ammonium nitrate, *etc.*; amino acids, such as glutamic acid, methionine, glutamine, *etc.*; and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* However, in one aspect of the present disclosure, diammonium adipate may be included as a single nitrogen source or as an additional nitrogen source.

The second step of converting lysine adipate into cadaverine adipate may be performed after the first step, in which lysine adipate is produced by culturing the L-lysine-producing microorganism in the medium containing diammonium adipate.

The second step refers to a step of converting lysine adipate into cadaverine adipate, which is the target product.

In one embodiment, the second step may comprise additionally adding adipic acid to the process liquid. The process liquid may be a fermentation process liquid obtained in the first step.

The adipic acid to be added in the second step may not be added or may be added in a trace amount of 0.6 mol/L or less, 0.35 mol/L or less, 0.18 mol/L or less, 0.1 mol/L or less, 0.08 mol/L or less, 0.05 mol/L or less, 0.04 mol/L or less, 0.03 mol/L or less, or 0.02 mol/L or less based on the volume of the process liquid, specifically, based on the volume of the fermentation process liquid obtained in the first step.

When adipic acid is additionally added to the process liquid in the second step, the adipic acid may be added before, after, or at the same time as the conversion reaction, but is not limited thereto.

When adipic acid is additionally added in the second step, it should be understood that the amount of adipate added during the entire process of the preparation method of the present disclosure is the sum of the amount of diammonium adipate added to the medium in the first step and the amount of adipic acid added in the second step. Additionally, when adipic acid is not additionally added to the process liquid in the second step, it should be understood that the amount of adipate added during the entire process of the preparation method of the present disclosure is the same as the amount of diammonium adipate added to the medium in the first step.

Specifically, the amount of adipate to be added in the preparation method of the present disclosure may be added in consideration of the amount of cadaverine in the process liquid to be obtained after the second step. Specifically, the adipate added in the preparation method of the present disclosure may be added at a molar ratio of 0.85 to 1.05 relative to the amount of cadaverine in the process liquid after the second step.

More specifically, the amount of adipate added in the preparation method of the present disclosure may be added at a molar ratio of 0.87 to 1.04, 0.87 to 1.03, 0.88 to 1.03, 0.88 to 1.02, 0.89 to 1.03, 0.89 to 1.02, 0.89 to 1.01 or 0.9 to 1.01 relative to the amount of cadaverine in the process liquid after the second step.

In one example, the amount of adipate added in the preparation method of the present disclosure may be contained at a molar ratio in the range consisting of one lower limit selected from 0.85, 0.86, 0.87, 0.88, 0.89, 0.9, 0.91, and 0.92 and/or one upper limit selected from 1.05, 1.04, 1.03, 1.02, and 1.01, relative to cadaverine in the process liquid after the second step.

In the preparation method of the present disclosure, there is a technical significance in proving that when adipate is added at a molar ratio within the above range relative to the amount of cadaverine calculated to be obtained after the second step, adipate does not precipitate as crystals together with cadaverine adipate, and thus cadaverine adipate can be prepared with high purity and high yield.

Additionally, cadaverine adipate can be prepared with a total yield of 45% or more, 47% or more, 57% or more, 59% or more, 64% or more, 68% or more, 69% or more, 70% or more, 71% or more, 72% or more, 73% or more, or 74% or more according to the number of circulation of the mother liquor, while maintaining cadaverine adipate at a purity of 99% up to three circulations, when circulating the mother liquor by adjusting the molar ratio of cadaverine to be obtained and adipate to be added.

In the second step, the conversion reaction of lysine adipate to cadaverine adipate may be an enzyme conversion reaction.

In one embodiment, the lysine adipate may comprise both the lysine adipate obtained in the first step and the lysine adipate produced in the second step when adipic acid is additionally added in the second step.

Specifically, the enzyme conversion reaction in the second step may be performed using a protein having lysine decarboxylase activity or a microorganism expressing a protein having the activity.

In one specific example, a protein having lysine decarboxylase activity may be added to the fermentation process liquid of the first step, or a seed culture medium of a microorganism expressing a protein having the above activity may be added, but is not limited thereto.

As used herein, the term "decarboxylase" is an enzyme that catalyzes the production of carbon dioxide by removing a carboxyl group from an organic acid, and may also be referred to as carboxy-lyase, and carbon-carbon lyase *etc.*

The protein is not particularly limited as long as it exhibits lysine decarboxylase activity, but in one example, it may be a *Pseudomonas thermotolerans*-derived PtLDC protein or an *Escherichia coli*-derived CadA protein. The protein sequence may be obtained using GenBank, a known database, the enzyme may be expressed using the above microorganisms, or a commercially available enzyme may be purchased for use, but is not limited thereto.

The microorganism expressing the protein may be a microorganism transformed to express the protein. The transformed microorganism is not limited to prokaryotic microorganisms and eukaryotic microorganisms as long as it is transformed to express the protein having decarboxylase activity. The microorganism transformed to express the protein having decarboxylase activity can excrete the protein having the enzyme activity into the seed culture medium, thereby converting lysine adipate in the process liquid into cadaverine adipate.

In one specific example, the microorganism may comprise those belonging to the genus *Escherichia,* the genus *Erwinia,* the genus *Serratia,* the genus *Providencia,* and Coryneform microbial strains. The microorganism may specifically be a microorganism belonging to the genus *Escherichia* or the genus *Corynebacterium,* and more specifically *Escherichia coli* or *Corynebacterium glutamicum,* but is not limited thereto.

The conversion reaction in the second step may be performed for 20 minutes to 3 hours, more specifically for 0.5 to 1.5 hours, and for example, may be performed for 1 hour, but is not limited thereto.

The conversion reaction in the second step may be performed at 30 to 60°C, more specifically at 40 to 50°C, and for example, may be performed at 45°C, but is not limited thereto.

The conversion reaction in the second step may be performed at a pH of 7.5 to 9, more specifically at 7.8 to 8.7, and even more specifically at 8 to 8.5. In one embodiment of the second step, the pH may be adjusted to the above range by adipic acid, which is selectively added in addition, and in another embodiment, the pH may be adjusted to the above range by adding $CO_2$. When the pH range of the conversion reaction in the second step is adjusted to the above range, the conversion rate of lysine adipate to cadaverine adipate can be significantly increased.

The method for preparing cadaverine adipate of the present disclosure may further comprise a recovering step of recovering the obtained cadaverine adipate after performing the second step.

In the recovering step, cadaverine adipate may be collected using a suitable method known in the art.

For example, methods such as centrifugation, filtration, concentration, crystallization, extraction, ultrasonic disruption, ultrafiltration, dialysis, various kinds of chromatography such as molecular sieve chromatography (gel filtration), adsorption chromatography, ion exchange chromatography, affinity chromatography, *etc.,* HPLC or a combination thereof may be used to collect cadaverine adipate.

In one embodiment of the present disclosure, the recovering step may comprise one or more of a filtration step, a concentration step, and a crystallization step.

The filtration step refers to a step of removing impurities from the conversion liquid containing cadaverine adipate obtained in the second step. In one example, the filtration may comprise removing microbial cells in the cadaverine adipate process liquid by performing membrane separation, *etc.,* or performing activated carbon filtration, *etc.*

The concentration step refers to a step of concentrating to increase the solid content ratio in the conversion liquid, and may be performed after the filtration step. In one example, the concentration may be reduced pressure concentration using a rotary evaporator.

Specifically, the concentration step may be performed such that the solid content is in the range of 65 to 85% (w/w), more specifically 70 to 85%(w/w), 72 to 83%(w/w), 65 to 80%(w/w), 65 to 75%(w/w), 75 to 85%(w/w), 70 to 80% (w/w), 72 to 78%(w/w), or 73 to 77%(w/w), for example, 75% (w/w).

The concentration step may be performed at 40 to 60°C, more specifically at 45 to 55°C, and for example, may be performed at 50°C, but is not limited thereto.

The crystallization step may be a cooling crystallization step, which means a step of cooling the conversion liquid and precipitating the liquid into crystals to obtain cadaverine adipate crystals, which are the final target product.

Specifically, the cooling may involve cooling the conversion liquid to 20 to 30°C, more specifically to 23 to 27°C, and in one example, it may be cooled to 25°C, but is not limited thereto.

Specifically, the cooling rate may be 5 to 20°C/hour, more specifically 5 to 15°C/hour, and in one example, it may be 10°C/hour, but is not limited thereto.

In one embodiment, the recovering step may further comprise a stirring step of stirring the process liquid. The stirring step may be performed simultaneously with the cooling crystallization step, in the middle of the cooling crystallization step, before the cooling crystallization step, or after the cooling crystallization step. Through the stirring step, time is given to sufficiently stir the process liquid, which facilitates crystal precipitation.

In one embodiment, the recovering step may further comprise a mother liquor circulation step of re-circulating the mother liquor containing cadaverine adipate that has not been crystallized after the crystallization step as the feed liquor of the concentration step.

Specifically, in the mother liquor circulation step, the number of circulations of the mother liquor may be 0 to 3 times, 1 to 3 times, 1 to 4 times, 1 to 5 times, 1 to 10 times, or more. In particular, the mother liquor circulation number of 0 means that the mother liquor circulation step is not performed.

Conventionally, in the crystallization step of cadaverine adipate, there was a problem in that relatively more than 50% of cadaverine adipate still remained in the mother liquor remaining after crystallization, resulting in a low yield of less than 50%. In the present disclosure, as a method of improving the yield of cadaverine adipate, the total yield of cadaverine adipate can be increased by 69% or more, 70% or more, 71% or more, 72% or more, 73% or more, 74% or more, or more by re-circulating the residual mother liquor of cadaverine adipate as the feed liquor of the concentration step for 1 to 3 times, 1 to 4 times, 1 to 5 times, and 1 to 10 times.

In one embodiment, the recovering step may further comprise the steps of washing, separating, and drying the crystallized process liquid.

In the drying step, the moisture contained in the crystals is removed through drying, and high purity cadaverine adipate can be commercialized. After drying, cadaverine adipate crystals can be provided in powder form, but the formulation may vary as needed.

In one embodiment, the recovering step may further comprise a decolorization step. The decolorization may be performed using activated carbon, anion resin, *etc.,* but is not limited thereto.

Additionally, the method for preparing cadaverine adipate of the present disclosure may comprise an additional purification step, which may be performed using an appropriate method known in the art. In one example, when the method for preparing cadaverine adipate of the present disclosure comprises both a recovering step and a purification step, the recovering step and the purification step may be performed continuously or intermittently regardless of the order or simultaneously, or may be integrated into one step, but the method is not limited thereto.

The preparation method of the present disclosure may be characterized in that it does not comprise a separate process for removing adipate in the recovery step. In the case of the conventional process for preparing cadaverine adipate, cadaverine adipate precipitated as crystals during the crystallization process, decreasing the purity, and in order to prevent the decrease, a separate process of removing adipate was required before crystallization. However, the preparation method of the present disclosure provides the technical advantage of preparing high-purity cadaverine adipate without performing a separate removal process for adipate.

In one embodiment, the method of the present disclosure may be characterized in that cadaverine adipate with a purity of 99% or more can be obtained in 0 to 3 circulations of the mother liquor.

In one embodiment, based on the three circulations of the mother liquor, the cumulative total yield of cadaverine adipate may be 65% or more, and more specifically 69%, 70%, 71%, 72%, 73%, or 74% or higher.

Through the method of the present disclosure, cadaverine adipate with high purity can be prepared without decarbonation and distillation processes required for conventional cadaverine liquid production.

## [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

In the above, the preparation process of cadaverine adipate according to one aspect of the present disclosure has been examined. Hereinafter, the beneficial effects mentioned in the present disclosure will be examined through the experimental results of Examples.

## Preparation Example 1-1. Fermentation Process for Lysine Adipate Preparation

The *Corynebacterium glutamicum* strain (KCCM12154P, US Publication No. US 2021-0355514 A1) with lysine-producing ability was obtained through solid-phase culture and flask culture to produce seeds, and then subjected to a seed culture in a fermenter, followed by fermentation. The fermentation was carried out via a fermenter culture at 36°C for 30 hours at 900 rpm.

Instead of ammonium sulfate, diammonium adipate was supplied at a level of 72 to 117 g/L for each example, and as a result of fermentation, lysine adipate at a level of 116 to 190 g/L was obtained, and lysine at a level of 99 g/L was obtained. In particular, in the Examples, the molar ratio of adipate /lysine in the fermentation process liquid was adjusted to a range of 0.59 to 0.96, and in particular in Examples 4 to 7, the molar ratio of adipate /lysine in the fermentation process liquid was adjusted to a range of 0.88 to 0.96.

## Preparation Example 1-2. Conversion Reaction of Lysine Adipate to Cadaverine Adipate

For the conversion reaction, *E. coli* (US Patent Publication US 2018-0030430 A1), in which the PtLDC enzyme, a lysine decarboxylase gene derived from *Pseudomonas thermotolerans,* was overexpressed using the pET-Deut1 vector, was used.

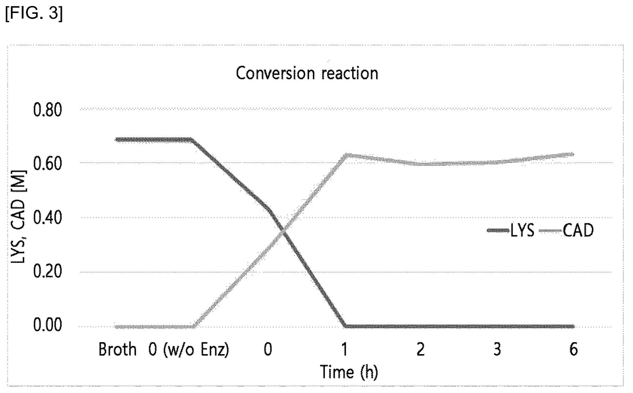

A conversion reaction was performed by supplying the seed culture medium of the enzyme strain at a level of 10% based on mass percentage to the fermentation process liquid of lysine adipate prepared in Preparation Example 1-1. The conversion reaction was carried out for about 1 hour while maintaining the temperature at 45 to 50°C. The pH was adjusted to 8.0 to 8.5, and in this regard, in Examples 1-4 below, $CO_2$ was added for neutralization, and in Examples 2 and 3, adipic acid was additionally added at a level of 0.02 to 0.03 mol/L (based on the volume of the fermentation process liquid of lysine adipate). FIG. 3 is a graph showing the results of the conversion reaction, and it was confirmed that the conversion rate was at the level of 97 to 98%. After the conversion reaction, cadaverine adipate was obtained at a level of 99 to 161 g/L (based on the volume of the fermentation process liquid for lysine adipate), and cadaverine was obtained at a level of 68 g/L (based on the volume of the fermentation process liquid for lysine adipate. Thereafter, in Examples 5-7, the process liquid was prepared by additionally adding adipic acid at a level of 0.02 to 0.15 mol/L.

## Preparation Example 1-3. Subsequent Processes After Obtaining Cadaverine Adipate

After the conversion reaction according to Preparation Example 1-2, the following subsequent processes were performed on the process liquid containing cadaverine adipate to obtain cadaverine adipate in crystal form.

### [Step of Removing Microbial Cells]

Microbial cells in the cadaverine adipate process liquid were removed by membrane filtration with a pore size of 0.1 $\mu$m.

### [Step of Removing Impurities using Activated Carbon]

Activated carbon was added at a level of 10% based on the weight of cadaverine adipate in the filtrate from which the microbial cells were removed, heated to 60°C, and stirred for 1 hour for decolorization, and then the activated carbon was filtered through filter paper.

### [Step of Concentrating Cadaverine Adipate Process Liquid from which Impurities have been Removed]

The filtrate, from which the activated carbon was removed, was concentrated under reduced pressure in a rotary evaporator at about 50°C and 120 Torr until the solid content reached 75% by weight.

### [Steps of Cooling Crystallizing and Separating Cadaverine Adipate Concentrate, followed by Drying]

The concentrate was cooled from 50°C to 25°C at a rate of 10°C/hr. The precipitated crystals were separated from the mother liquor using a centrifuge. The crystals were washed with 10% water by weight of cadaverine adipate. The separated crystals were dried for a day and then the purity was measured by HPLC.

### [Step of Re-circulating Mother Liquor into Crystallization Step]

The mother liquor separated above was re-circulated to [the step of concentrating the cadaverine adipate process liquid from which impurities were removed].

## Experimental Example 1. Confirmation of Changes in Purity and Yield of Cadaverine Adipate According to Molar Ratio of Adipate and Cadaverine

In Experimental Example 1, the method according to Preparation Examples 1-1 to 1-3 was used, and it was confirmed how the purity and yield of cadaverine adipate changed depending on the molar ratio of adipate added during the entire process and cadaverine in the process liquid after the second step.

**Example 1: When the molar ratio of adipate added/cadaverine in the process liquid after the second step is 0.6**

1,000 ml of cadaverine adipate conversion liquid with an adipate/cadaverine molar ratio of 0.6 was passed through a membrane with a pore size of 0.1 $\mu$m to remove microbial cells. The cadaverine adipate conversion liquid with an adipate /cadaverine molar ratio of 0.6 was prepared such that the total of diammonium adipate added in the fermentation process to produce lysine adipate and adipic acid added in the cadaverine adipate conversion reaction may be 0.6 of the number of moles of converted cadaverine (refer to Formula 1 below).

[Formula 1]

$$\frac{Total\ of\ adipate(mole)of\ diammonium\ adipate\ added\ in\ the\ fermentation\ process\ to\ produce\ lysine\ adipate(Step\ 1)and\ adipic\ acid(mole)added\ in\ the\ cadaverine\ adipate\ conversion\ reaction}{converted\ cadaverine(mole)}$$

The filtrate, from which microbial cells had been removed, was decolorized using activated carbon, filtered through filter paper, and then concentrated under reduced pressure to a solid content of 75%. The concentrate was crystallized by cooling from 60°C to 25°C. The crystals and mother liquor were separated through centrifugation, and the separated crystals were dried for a day, and then the purity was measured by HPLC. The mother liquor, in which adipate remained, was re-circulated as the feed liquor for cadaverine adipate with an adipate/cadaverine molar ratio of 0.6, and then the crystallization step was performed again. The recirculation of the mother liquor was carried out sequentially.

**Example 2: When the molar ratio of adipate added/cadaverine in the process liquid after the second step is 0.71**

A cadaverine adipate conversion liquid with an adipate /cadaverine molar ratio of 0.71 was supplied, and the purification process was performed in the same manner as in Example 1 while sequentially re-circulating the mother liquor.

**Example 3: When the molar ratio of adipate added/cadaverine in the process liquid after the second step is 0.8**

A cadaverine adipate conversion liquid with an adipate /cadaverine molar ratio of 0.8 was supplied, and the purification process was performed in the same manner as in Example 1 while sequentially re-circulating the mother liquor.

**Example 4: When the molar ratio of adipate added/cadaverine in the process liquid after the second step is 0.9**

A cadaverine adipate conversion liquid with an adipate /cadaverine molar ratio of 0.9 was supplied, and the purification process was performed in the same manner as in Example 1 while sequentially re-circulating the mother liquor.

**Example 5: When the molar ratio of adipate added/cadaverine in the process liquid after the second step is 1.01**

A cadaverine adipate conversion liquid with an adipate /cadaverine molar ratio of 1.01 was supplied, and the purification process was performed in the same manner as in Example 1 while sequentially re-circulating the mother liquor.

In the case of Examples 4 and 5, the molar ratio of adipate /lysine in the lysine fermentation step was adjusted to 0.85 to 0.98.

**Example 6: When the molar ratio of adipate added/cadaverine in the process liquid after the second step is 1.1**

Cadaverine adipate conversion liquid with an adipate /cadaverine molar ratio of 1.1 was supplied, and purification process was performed in the same manner as in Example 1 while sequentially re-circulating the mother liquor.

**Example 7: When the molar ratio of adipate added/cadaverine in the process liquid after the second step is 1.2**

Cadaverine adipate conversion liquid with an adipate /cadaverine molar ratio of 1.2 was supplied, and purification process was performed in the same manner as in Example 1 while sequentially re-circulating the mother liquor.

The measured physical properties of the process liquids and cadaverine adipate prepared according to the above-mentioned Examples 1 to 7 are shown in Tables 1 to 4 below.

Table 1 is for the process liquid without circulation of mother liquor.

[Table 1]

| No Circulation of Mother Liquor | Example 1 | Example 2 | Example 3 | Example 4 | Example 5 | Example 6 | Example 7 |
|---|---|---|---|---|---|---|---|
| Molar Ratio of Adipate /Cadaverine | 0.6 | 0.71 | 0.8 | 0.9 | 1.01 | 1.1 | 1.2 |
| Crystallization Feed Liquor | | | | | | | |
| New Feed Liquor, ml | 1000 | 1000 | 1000 | 1000 | 1000 | 1000 | 1000 |
| Amount of Cadaverine in Crystallization Liquid, g | 285 | 285 | 285 | 285 | 285 | 285 | 285 |
| Amount of Cadaverine in Produced Crystals, g | No crystals formed | 107 | 118 | 127 | 134 | 125 | 116 |
| Amount of Cadaverine in Mother Liquor, g | - | 178 | 167 | 158 | 151 | 160 | 169 |
| Results | | | | | | | |
| Purity, % | - | 98% | 98% | 98% | 99% | 97% | 92% |
| Yield, % | - | 38% | 41% | 45% | 47% | 44% | 41% |
| Total Yield, % | - | 38% | 41% | 45% | 47% | 44% | 41% |

Table 2 is for the process liquid with circulation of mother liquor for 1 time.

[Table 2]

| Circulation of Mother Liquor for 1 time | Example 1 | Example 2 | Example 3 | Example 4 | Example 5 | Example 6 | Example 7 |
|---|---|---|---|---|---|---|---|
| Molar Ratio of Adipate /Cadaverine | 0.6 | 0.71 | 0.8 | 0.9 | 1.01 | 1.1 | 1.2 |
| Crystallization Feed Liquor | | | | | | | |
| New Feed Liquor, ml | | 1000 | 1000 | 1000 | 1000 | 1000 | 1000 |
| Amount of Cadaverine in Crystallization Liquid, g | | 462 | 451 | 443 | 436 | 445 | 453 |
| Amount of Cadaverine in Produced Crystals, g | | 174 | 187 | 197 | 204 | 195 | 184 |
| Amount of Cadaverine in Mother Liquor, g | | 289 | 264 | 246 | 231 | 250 | 269 |

(continued)

| Results | | | | | | | |
|---|---|---|---|---|---|---|---|
| Purity, % | | 98% | 99% | 99% | 99% | 93% | 84% |
| Yield, % | | 38% | 41% | 45% | 47% | 44% | 41% |
| Total Yield, % | | 49% | 54% | 57% | 59% | 56% | 53% |

Table 3 is for the process liquid with circulation of mother liquor for 2 times.

[Table 3]

| Circulation of Mother Liquor for 2 times | Example 1 | Example 2 | Example 3 | Example 4 | Example 5 | Example 6 | Example 7 |
|---|---|---|---|---|---|---|---|
| Molar Ratio of Adipate /Cadaverine | 0.6 | 0.71 | 0.8 | 0.9 | 1.01 | 1.1 | 1.2 |
| Crystallization Feed Liquor | | | | | | | |
| New Feed Liquor, ml | - | 1000 | 1000 | 1000 | 1000 | 1000 | - |
| Amount of Cadaverine in Crystallization Liquid, g | - | 573 | 549 | 530 | 516 | 534 | - |
| Amount of Cadaverine in Produced Crystals, g | - | No crystals formed | 206 | 226 | 242 | 221 | - |
| Amount of Cadaverine in Mother Liquor, g | - | - | 342 | 305 | 274 | 313 | - |
| Results | | | | | | | |
| Purity, % | - | - | 98% | 98% | 99% | 86% | - |
| Yield, % | - | - | 38% | 43% | 47% | 41% | - |
| Total Yield, % | - | - | 60% | 64% | 68% | 63% | - |

Table 3 is for the process liquid with circulation of mother liquor for 3 times.

[Table 4]

| Circulation of Mother Liquor for 3 times | Example 1 | Example 2 | Example 3 | Example 4 | Example 5 | Example 6 | Example 7 |
|---|---|---|---|---|---|---|---|
| Molar Ratio of Adipate /Cadaverine | 0.6 | 0.71 | 0.8 | 0.9 | 1.01 | 1.1 | 1.2 |
| Crystallization Feed Liquor | | | | | | | |
| New Feed Liquor, ml | - | - | 1000 | 1000 | 1000 | - | - |
| Amount of Cadaverine in Crystallization Liquid, g | - | - | 285 | 675 | 627 | - | - |
| Amount of Cadaverine in Produced Crystals, g | - | - | No crystals formed | 265 | 294 | - | - |
| Amount of Cadaverine in Mother Liquor, g | - | - | - | 409 | 333 | - | - |

(continued)

| Results | | | | | | | |
|---|---|---|---|---|---|---|---|
| Purity, % | - | - | - | 99% | 99% | - | - |
| Yield, % | - | - | - | 39% | 47% | - | - |
| Total Yield, % | - | - | - | 69% | 74% | - | - |

In summary of the experimental results according to Tables 1 to 4 above, it was confirmed that it was possible to produce cadaverine adipate with improved crystallization rate, high purity, and high yield through the preparation method of the present disclosure, in which diammonium adipate in the first step was contained in large amounts at a level of 81 to 100 mol% of the total adipate supplied.

More specifically, in Example 1, crystals did not precipitate even after the crystallization process. This is because the molar ratio of adipate was low, which was not enough for crystals of cadaverine adipate to precipitate.

In Example 2, crystals precipitated until the first circulation of mother liquor, but no crystals were formed during the second circulation. This is because the molar ratio of adipate in the crystallization feed liquor was lowered after the first circulation of mother liquor, which was not enough for crystals of cadaverine adipate to precipitate.

In Example 3, crystals precipitated until the second circulation of mother liquor, but no crystals were formed during the third circulation. This is because the sequentially circulated mother liquor lowered the molar ratio of adipate to the crystallization feed liquid, and accordingly, the concentration of adipate was not sufficient to precipitate crystals of cadaverine adipate.

In Example 4, cadaverine adipate with high purity of 99% was obtained until the third circulation of mother liquor, and a total yield of about 70% was achieved, which is a high yield.

In Example 5, cadaverine adipate with high purity of 99% was obtained until the third circulation of mother liquor, and a total yield of about 74% was achieved, which is a high yield.

In Example 6, crystals precipitated until the second circulation of mother liquor, but the purity of the crystals was reduced slightly to 86%. This is because adipate accumulated in the crystallization feed liquid as the mother liquor was sequentially circulated, and adipic acid was precipitated as an impurity.

In Example 7, the purity of the crystals was reduced slightly to 84% in the first circulation of mother liquor. This is because adipate accumulated in the crystallization feed liquid as the mother liquor was sequentially circulated, and adipic acid was precipitated as an impurity.

As such, it was found that the method for preparing high purity cadaverine adipate can be provided depending on the molar ratio of adipate added during the entire process relative to the amount of cadaverine in the process solution after the second step. In particular, when the amount of adipate added is adjusted to a molar ratio of about 0.9 to 1.01 relative to the amount of cadaverine in the process liquid after the second step, it was confirmed that an excellent total yield of 69% or more could be achieved, while maintaining cadaverine adipate at a purity of 99% or more even with three or more circulations of the mother liquor.

From the foregoing, a skilled person in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

**Claims**

1. A method for preparing cadaverine adipate, comprising:

   a first step of obtaining lysine adipate by culturing an L-lysine-producing microorganism in a medium to which adipate is added in the form of diammonium adipate; and
   a second step of converting lysine adipate into cadaverine adipate, wherein the amount of adipate added is contained in a molar ratio of 0.85 to 1.05 compared to the amount of cadaverine in the process solution after the second step.

2. The method of claim 1, wherein the diammonium adipate in the first step is contained in an amount of 81 mol% or more of the total adipate supplied.

3. The method of claim 1, wherein the diammonium adipate in the first step is contained such that the molar ratio of lysine to be produced (diammonium adipate/lysine) is 0.85 to 0.98.

4. The method of claim 1, wherein the conversion reaction in the second step is carried out at a pH of 8.0 to 8.5.

5. The method of claim 1, wherein the amount of adipate added is contained at a molar ratio of 0.88 to 1.02 compared to the amount of cadaverine in the process solution after the second step.

6. The method of claim 1, further comprising a recovery step of recovering cadaverine adipate after performing the second step.

7. The method of claim 6, wherein the recovery step comprises at least one step selected from the group consisting of a filtration step, a concentration step, and a cooling crystallization step.

8. The method of claim 7, wherein the recovering step further comprises a mother liquor circulation step of re-circulating the mother liquor containing cadaverine adipate that has not been crystallized after the cooling crystallization step as the feed solution of the concentration step.

9. The method of claim 7, wherein the concentration step is performed such that the solid content is 70 to 80% (w/w).

10. The method of claim 1, wherein in the second step, the conversion of lysine adipate to cadaverine adipate is performed using a protein having lysine decarboxylase activity or a microorganism expressing a protein having the activity.

11. The method of claim 1, wherein the L-lysine-producing microorganism is *Corynebacterium glutamicum.*

12. The method of claim 1, wherein the recovering step does not comprise a separate process for removing adipate in the process solution.

13. The method of claim 8, wherein cadaverine adipate with a purity of 99% or more is obtained in 0 to 3 times of mother liquor circulation.

[FIG. 1]

Start

Obtaining lysine adipate by culturing L-lysine-producing microorganism in a medium containing diammonium adipate

Converting obtained lysine adipate into cadaverine adipate

End

[FIG. 2]

[FIG. 3]

## INTERNATIONAL SEARCH REPORT

| International application No. |
| --- |
| **PCT/KR2024/006740** |

| A. | CLASSIFICATION OF SUBJECT MATTER |
| --- | --- |

**C12P 13/00**(2006.01)i; **C12P 13/08**(2006.01)i; **C12N 15/77**(2006.01)i; **C12N 9/88**(2006.01)i

According to International Patent Classification (IPC) or to both national classification and IPC

| B. | FIELDS SEARCHED |
| --- | --- |

Minimum documentation searched (classification system followed by classification symbols)

C12P 13/00(2006.01); C07C 211/09(2006.01); C08G 69/26(2006.01); C08G 69/28(2006.01); C12N 1/32(2006.01); C12N 15/09(2006.01); C12N 9/00(2006.01); C12P 13/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 디암모늄 아디핀산염(diammonium adipate), L-라이신(L-lysine), 라이신 아디핀산염(lysine adipate), 카다베린 아디핀산염(cadaverine adipate), 코리네박테리움 글루타미쿰(corynebacterium glutamicum)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | EP 1482055 A1 (AJINOMOTO CO., INC.) 01 December 2004 (2004-12-01)<br>See claims 1, 4 and 5; and paragraphs [0065]-[0068]. | 1-13 |
| A | KR 10-2022-0153598 A (MOJIA BIOTECH LTD.) 18 November 2022 (2022-11-18)<br>See claims 1 and 7. | 1-13 |
| A | KR 10-2009-0005099 A (BASF SE) 12 January 2009 (2009-01-12)<br>See claims 1 and 8. | 1-13 |
| A | JP 2008-189661 A (MITSUBISHI CHEMICALS CORP.) 21 August 2008 (2008-08-21)<br>See claims 1-14. | 1-13 |
| A | WO 2015-195703 A1 (INVISTA TECHNOLOGIES S.A.R.L.) 23 December 2015 (2015-12-23)<br>See claims 1-14. | 1-13 |

☐ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **29 August 2024** | **30 August 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT
### Information on patent family members

International application No.

**PCT/KR2024/006740**

| Patent document cited in search report | | | Publication date (day/month/year) | Patent family member(s) | | | Publication date (day/month/year) |
|---|---|---|---|---|---|---|---|
| EP | 1482055 | A1 | 01 December 2004 | EP | 1482055 | B1 | 01 March 2006 |
| | | | | US | 2005-0003497 | A1 | 06 January 2005 |
| | | | | US | 7189543 | B2 | 13 March 2007 |
| KR | 10-2022-0153598 | A | 18 November 2022 | CN | 113122589 | A | 16 July 2021 |
| | | | | CN | 115181763 | A | 14 October 2022 |
| | | | | EP | 4118224 | A1 | 18 January 2023 |
| | | | | JP | 2023-518223 | A | 28 April 2023 |
| | | | | US | 2023-0110819 | A1 | 13 April 2023 |
| | | | | WO | 2021-185191 | A1 | 23 September 2021 |
| KR | 10-2009-0005099 | A | 12 January 2009 | CN | 101389765 | A | 18 March 2009 |
| | | | | CN | 101389765 | B | 22 August 2012 |
| | | | | CN | 102732578 | A | 17 October 2012 |
| | | | | CN | 102732578 | B | 17 February 2016 |
| | | | | EP | 2013353 | A1 | 14 January 2009 |
| | | | | EP | 2013353 | B1 | 22 June 2016 |
| | | | | JP | 2009-531042 | A | 03 September 2009 |
| | | | | JP | 2013-146269 | A | 01 August 2013 |
| | | | | JP | 5210295 | B2 | 12 June 2013 |
| | | | | JP | 5745552 | B2 | 08 July 2015 |
| | | | | KR | 10-1457229 | B1 | 04 November 2014 |
| | | | | US | 2009-0246838 | A1 | 01 October 2009 |
| | | | | US | 8741623 | B2 | 03 June 2014 |
| | | | | WO | 2007-113127 | A1 | 11 October 2007 |
| JP | 2008-189661 | A | 21 August 2008 | JP | 5365004 | B2 | 11 December 2013 |
| WO | 2015-195703 | A1 | 23 December 2015 | CN | 106795532 | A | 31 May 2017 |
| | | | | EP | 3155090 | A1 | 19 April 2017 |
| | | | | US | 2015-0361464 | A1 | 17 December 2015 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20210355514 A1

- US 20180030430 A1